# EUROPEAN PATENT APPLICATION

(11) **EP 4 242 705 A1**
(43) Date of publication of application: **13.09.2023**
(21) Application number: 22160835.9
(22) Date of filing: 08.03.2022
(51) Int. Cl.: G02B 1/04

(54) **POLYMER ARTICLES FOR OPHTHALMIC PRODUCTS, AND METHOD FOR REDUCING CALCIFICATION PROPENSITY OF POLYMERS**

(71) Applicant: ACTIOL GmbH, 35287 Amöneburg (DE)
(72) Inventor: KLEIN, Felix, 55130 Mainz (DE); HEE-CHEOL, Kim, 35039 Marburg (DE)
(74) Representative: Fuchs Patentanwälte Partnerschaft mbB

(57) **Abstract**

A method for reducing the calcification propensity of polymer articles is disclosed. The method comprises effecting a blueish fluorescence of the polymers by irradiation with UV light and/or heating the polymers, in particular hydrophilic polymers for intraocular lenses.

## Description

The present invention relates to calcification propensity of polymer articles, in particular hydrophilic polymer articles for intraocular lenses, and provides a method for reducing the same.

### Prior art

A common problem with polymers used in optical lenses for the human body is their tendency to opacify during the wearing time. This occurs in particular with intraocular lenses (IOL). The cause for the opacification is the formation of calcific deposits on the surface and in the surface layers up to a depth of about 40 µm (different crystal forms of calcium phosphate). The phenomenon is known in the art under the term "calcification". Hydrophilic polymers are more prone to calcification than hydrophobic polymers.

The IOLs show calcification *in vivo* to such a degree that the artificial eye lens becomes cloudy and the implant needs to be replaced to restore vision. P. Kanclerz et al., Curr. Opin. Ophthalmol. (2021), 32, 31-44 and A. Grzybowski et al., Ann. Transl. Med. (2020), 8, 1547-1547 have found that the incidence for this is < 1 % of all implanted hydrophilic IOLs, regardless of the manufacturer, but some batches of certain manufacturers show a significantly higher risk of (nano-)calcification.

Although the probability of (nano-)calcification impairing vision is clearly in the per mill range, hydrophilic intraocular lenses are used less and less. However, the hydrophilic polymers containing from 15 wt.-% up to nearly 50 wt.-% of water more strongly resemble the natural lens of the eye and, hence, are better biocompatible than hydrophobic polymers. In addition to the decreased biocompatibility, the latter also often tend to glistening, which causes a severe decay of the visual acuity.

The research in this art sector conducted until to date has provided the insight that the tendency towards calcification depends primarily on two factors, which are the patient and the IOL material. Regarding the patient, it has been generally agreed that diabetes is one important factor, since diabetes causes an increased porosity of the blood vessels resulting in increased levels of Ca²⁺ and phosphate ions. Further, there might be a calcification promoting effect on implanted IOLs of air cushions used in corneal transplantation.

In the prior art, there have been different approaches for eliminating or at least further minimizing the calcification since for restoring vision, the patient will inevitably have to undergo a further uncomfortable eye surgery which is associated with considerable costs and cannot be repeated limitless due to increasing difficulties with positioning and fixing the lenses.

In the case of batches with frequent calcifications, A. Grzybowski *et a*/*.* reported that the risk could already be reduced to a normal level by modifying the packaging process, in particular by eliminating silicone-containing material.

Furthermore, the general use of highly pure starting materials in polymerization is believed to be able to effect a reduction in the risk of developing calcifications. Another approach to counteract calcifications has been a hydrophobic surface modification of the lenses. However, J. F. Costa et al., Eur. J. Ophthalmol. (2020), 30, 307-314 and S. P. Gartaganis et al., Am. J. Ophthalmol. (2016), 168, 68-77 reported that in this case there was even an increased incidence of calcifications.

For example, US 2017/0172728 A1 discloses an IOL which is prepared by forming a hydrophobic poly-p-xylylene skin by means of a chemical vapor deposition (CVD) process which encapsulates a liquid hydrophilic core. Hence, the process is very complicated and requires expensive devices. Moreover, the optical quality of the lenses is difficult to ascertain due to the encapsulation process of the liquid core wherein a drop of the liquid is placed on the first CVD created poly-p-xylylene skin and the second poly-p-xylylene skin is deposited directly on the liquid. P-Xylylene is applied in vacuum and causes a hydrophobic surface but before implantation the hydrophilic material needs to take up considerable amounts of water to reach the most common 20-30 wt.-% of water.

Albeit promising better biocompatibility due to their high water content, the persisting problem of calcifications causes that hydrophilic IOLs are increasingly substituted by hydrophobic IOLs.

For the production of hydrophilic IOLs, there is an urgent need to have materials and processes available that significantly further reduce the risk of postoperative (nano-)calcification.

At present, there is no method available by which the occurrence of calcifications in intraocular lenses in general and hydrophilic IOLs in particular can be reliably reduced. There is not even an established test to quantify the risk of (nano-)calcification of IOLs on a batch level.

### Object

Hence, it is an object of the present invention to provide a method for the reduction of the calcification propensity of polymer articles. In particular, the method should not require the addition of further chemicals to the polymer or expensive equipment. Further, the method is to work on the polymer article and should be able to process a huge number of articles, e.g. IOLs, in short time. Finally, an article, particularly an ophthalmic medical aid, with reduced calcification propensity is to be provided.

### Description of the invention

These objects are achieved by a method, an article and use according to the independent claims. Preferred embodiments are the subject matter of the dependent claims.

Features of the polymer and article achieved by the method according to the invention are also applicable to the claimed article since the polymers of the article are the same.

Within this application, the polymer article comprises a "bulk volume" and a "surface layer". The terms are referring to the polymer volume of the article between its external surfaces, wherein external surfaces are referring to the interface between polymer and surrounding atmosphere and not external surfaces of hollow bodies. In a cross-section of the article or article walls, the "surface layers" are extending from the surfaces 300 µm into the article and surround the bulk volume.

The method for reducing the calcification propensity of an article, optionally shaped as an optical lens, an intraocular lens, or a button, or a preform of an optical or intraocular lens, comprises generating one or more fluorescent structures in or on the article, wherein the article comprises one or more polymers, wherein one or more of the polymers comprises an acrylic structural motive. The polymers are in particular biocompatible polymers.

The term "calcification propensity" is referring to the degree of calcification which is determined by an incubation test of 90 days at 35 °C in incubation solution L3 (see below) and is defined as the ratio between the area of calcific deposits on or in the article within the measured area and the whole measured area. Both terms are used interchangeably within this application.

An acrylic structural motive, as used within this application, is referring to repeating units of polymers which are based on a monomer of substituted or unsubstituted (meth)acrylic acid or their esters and amides.

Generating one or more fluorescent structures may comprise partially or completely irradiating the article with UV radiation, preferably having a wavelength of 220 nm - 380 nm, and/or heating the article above the glass transition temperature Tg of the polymer.

The inventors have surprisingly discovered that polymers used for IOLs may be treated using only physical means in such a way that the polymer, which itself is not fluorescent, develops a clear fluorescence. And it has been shown in *in vitro* stress tests that the propensity to calcification after performing this process is significantly reduced. The process according to the invention is particularly effective with hydrophilic polymers, which are typically used for ophthalmic medical aids, but also works with the corresponding hydrophobic polymers. Cyclic hydrocarbon structures within (intramolecular) or between (intermolecular) the polymer chains generated by a thermal treatment or an irradiation may be responsible for the fluorescence. The polymer may be used as a raw product, i.e. a preform (already in lens shape but not yet formed into the required optical shape) or a so called "button" or "round blank" (a chip-like shape), if a thermal treatment is conducted, or as a finished IOL, if an irradiation treatment is conducted.

The reason for this difference is that thermal treatment acts on the bulk material whereas irradiation treatment acts only on a surface layer. The limitation of the action of an irradiation treatment with UV light to the surface layer is caused by the addition of UV absorbers to the polymer which is required by medical regulations for IOLs. The term "UV absorbers" is referring to chemicals or compounds which absorb UV radiation and, hence, reduce the transmission of UV radiation through the IOLs in the UV-A, UV-B, and/or UV-C range. Examples of used UV absorbing compounds are 4-(2-acryloxyethoxy)-2-hydroxybenzophenone (AEHB) and 2-(4-benzoyl-3-hydroxyphenoxy)ethyl acrylate. Hence, depending on the irradiation time the fluorescence effect occurs up to a depth of 50-200 µm.

For the first time, the invention describes a process through which the occurrence of calcification can be demonstrably reduced *in vitro.*

Two examples for a reaction that leads to a cyclic structure in an acrylate polymer are shown in the reaction schemes below.

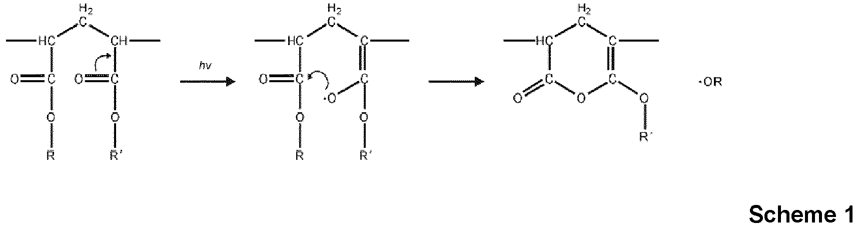

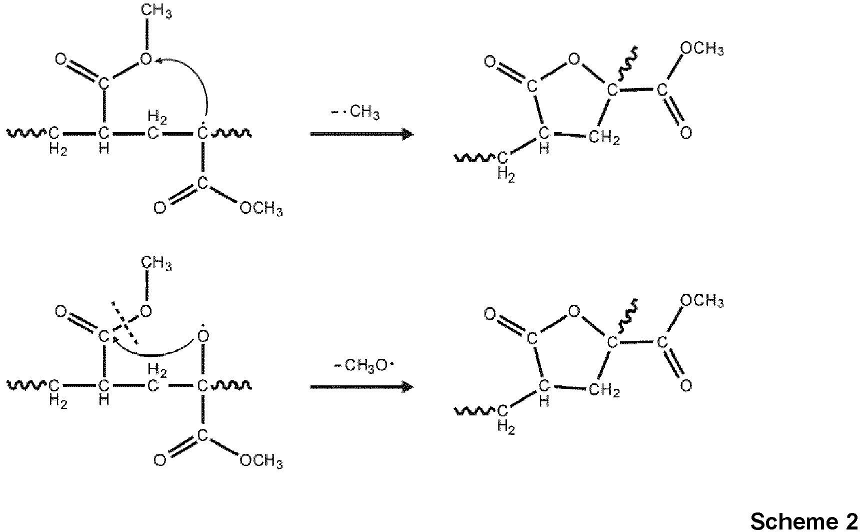

No oxygen is required for these reactions. They are photo-induced radical reactions which result in a cyclic product which fluoresces. A particularly typical structure which can be found in the fluorescing polymers is a lactone structure, i.e. the presence of cyclic carboxylic esters, containing a 1-oxacycloalkan-2-one structure, or analogues having unsaturation or heteroatoms, in particular nitrogen, replacing one or more carbon atoms of the ring. Examples of such lactone structures are also shown in above Schemes 1 and 2.

The method according to the invention for reducing the calcification propensity of polymer articles for the production of polymeric optical lenses comprises generating one or more fluorescent structures. Generating one or more fluorescent structures may comprise generating a fluorescent structure in the polymer, particularly by intramolecularly and/or intermolecularly condensing substituents and/or structural units of the polymer chain. This may eventually be followed by further reactions. In particular, this can be done without adding a chemical.

The fluorescent structure may be generated in the form of a separate substance or structures within the polymer. In particular, the fluorescent structure may be non-extractable from the polymer article. In the context of this invention, a "non-extractable" structure or substance is referring to a structure or substance which cannot be extracted by an extraction method as used for the determination of the extractables content according to DIN EN ISO 11979-5:2010-11, Appendix A, using methanol as the solvent. That is, the result of an extraction test before and after a treatment according to the invention remains the same. Since an extract of the polymer article does not contain any fluorescent compounds, the inventors believe that at least the majority of the fluorescence is generated by structures within the polymer chains.

A huge advantage of the method according to the invention is that no chemicals whatsoever have to be added into the polymer in order to reduce calcification propensity. Such chemicals might later, when the lens is worn by a patient, migrate out of it and cause irritation to the eye.

Another huge advantage is that, since no chemicals are added and the polymers remain essentially the same, no new approval process for the lenses has to be fulfilled which would be associated with extremely high costs.

Calcification of the polymer article may be quantified by the following incubation test procedure as an *in vitro* calcification test.

The articles to be tested, e.g. blanks or IOLs, are placed in a test cuvette with an incubation solution selected from L1-L4 (see Table 1) and incubated at 35 °C under exclusion of light. Optionally, incubation solution L4 is used because it has the highest concentration and, hence, requires the shortest incubation times to show a result. The other solutions may be selected if subtle differences between similar performing samples are to be investigated.

**Table 1**

| label | pH-value | **Ca²⁺** / **mM** | **PO₄**^{**3**-} / **mM** | albumin **/ mg/L** | **NaCl** / **mM** | **NaN₃ / ppm** |
|---|---|---|---|---|---|---|
| L1 | 7.00 | 1.45 | 0.23 | 100 | 0 | 100 |
| L2 | 7.00 | 1.45 | 0.32 | 250 | 0 | 100 |
| L3 | 7.00 | 2.50 | 1.05 | 250 | 0 | 100 |
| L4 | 7.00 | 4.00 | 2.40 | 250 | 140 | 100 |

The sample is measured at regular intervals by a measuring device comprising a white light source behind the sample and an electronic camera positioned in front of the sample. The camera records a focus stack of images, which is processed into an image with a high depth of focus.

This image is further processed and divided into clouded areas (recognizable by the white reflection) and transparent areas. All areas having a transparency below 95 % are considered to be calcified. In the measurements, no distinction is made between superficial calcifications and those in the material.

As a degree of calcification, the ratio of the calcified area divided by the total measuring area is calculated. It may with other words also be described as the percentage of sample area which is opacified by calcific deposits. Consequently, the reduction of the degree of calcification can be calculated by dividing the degree of calcification of a treated sample by that of an untreated sample and subtracting the result from 1.

In embodiments, the calcification propensity of the polymer is reduced by at least 50 % as compared to before treatment. For determining the reduction in calcification propensity, the degree of calcification of the polymer may be determined using solution L3 at 35 °C and an incubation time of 90 days in above mentioned incubation test procedure. Preferably, the calcification propensity is reduced by at least 55 % or by at least 60 % or by at least 65 % or by at least 70 % or by at least 75 % or by at least 80 % or by at least 85 % or by at least 90 % or by at least 95 % or by at least 99 % or by at least 99.5 %. The calcification propensity may also be reduced by 100 %. The calcification propensity of the polymer may be reduced by at most 55 % or by at most 60 % or by at most 65 % or by at most 70 % or by at most 75 % or by at most 80 % or by at most 85 % or by at most 90 % or by at most 95 % or by at most 99 % or by at most 99.5 %. The calcification propensity of the polymer may be reduced by 50 % - 100 % or by 50 % - 99.5 % or by 50 % - 99 % or by 50 % - 95 % or by 50 % - 90 % or by 50 % - 85 % or by 50 % - 80 % or by 50 % - 75 % or by 50 % - 70 % or by 50 % - 65 % or by 50 % - 60 % or by 50 % - 55 %. The calcification propensity of the polymer may be reduced by 55 % - 100 % or by 55 % - 99.5 % or by 55 % - 99 % or by 55 % - 95 % or by 55 % - 90 % or by 55 % - 85 % or by 55 % - 80 % or by 55 % - 75 % or by 55 % - 70 % or by 55 % - 65 % or by 55 % - 60 %. The calcification propensity of the polymer may be reduced by 60 % - 100 % or by 60 % - 99.5 % or by 60 % - 99 % or by 60 % - 95 % or by 60 % - 90 % or by 60 % - 85 % or by 60 % - 80 % or by 60 % - 75 % or by 60 % - 70 % or by 60 % - 65 %. The calcification propensity of the polymer may be reduced by 65 % - 100 % or by 65 % - 99.5 % or by 65 % - 99 % or by 65 % - 95 % or by 65 % - 90 % or by 65 % - 85 % or by 65 % - 80 % or by 65 % - 75 % or by 65 % - 70 %. The calcification of the polymer may be reduced by 70 % - 100 % or by 70 % - 99.5 % or by 70 % - 99 % or by 70 % - 95 % or by 70 % - 90 % or by 70 % - 85 % or by 70 % - 80 % or by 70 % - 75 %. The calcification propensity of the polymer may be reduced by 75 % - 100 % or by 75 % - 99.5 % or by 75 % - 99 % or by 75 % - 95 % or by 75 % - 90 % or by 75 % - 85 % or by 75 % - 80 %. The calcification propensity of the polymer may be reduced by 80 % - 100 % or by 80 % - 99.5 % or by 80 % - 99 % or by 80 % - 95 % or by 80 % - 90 % or by 80 % - 85 %. The calcification propensity of the polymer may be reduced by 85 % - 100 % or by 85 % - 99.5 % or by 85 % - 99 % or by 85 % - 95 % or by 85 % - 90 %. The calcification propensity of the polymer may be reduced by 90 % - 100 % or by 90 % - 99.5 % or by 90 % - 99 % or by 90 % - 95 %. The calcification propensity of the polymer may be reduced by 95 % - 100 % or by 95 % - 99.5 % or by 95 % - 99 %. The calcification propensity of the polymer may be reduced by 99 % - 100 % or by 99 % - 99.5 %. The calcification propensity of the polymer may be reduced by 99.5 % - 100 %. Particularly preferred ranges of the reduction of the calcification propensity are for example 50 % - 100 %, 60 % - 95 %, or 70 % - 90 %.

A polymer article treated according to the invention preferably has a calcification propensity (determined in an incubation test procedure as described above with solution L3 at 35 °C for 90 days) of less than 10 % or less than 9 % or less than 8 % or less than 7 % or less than 6 % or less than 5 % or less than 4 % or less than 3 % or less than 2 % or less than 1 %. Notably, the calcification test described herein is much harsher than the physiological conditions, i.e. if calcification is low in this test, it can be assumed that no calcification will occur under physiological conditions.

Treatment of the polymer according to the invention by UV irradiation will increase the fluorescence intensity with treatment time up to a maximum value. The maximum value is specific to the article. It is determined by continuing UV irradiation until no further increase can be obtained. "No further increase" is referring to a difference of less than 3 % between two measurements of the fluorescence intensity (in arbitrary units, a. u.) with a fluorescence spectrometer using an excitation wavelength of 365 nm. Once this maximum value is reached, a further treatment will not further enhance the fluorescence intensity. In embodiments, generating one or more fluorescent structures is performed such that the fluorescence intensity of the article is at least 80 % of the maximum intensity achievable in the article by UV irradiation. The treatment may preferably be carried out for a time sufficient to reach at least 85 % of the maximum intensity or at least 86 % or at least 87 % or at least 88 % or at least 89 % or at least 90 % or at least 91 % or at least 92 % or at least 93 % or at least 94 % or at least 95 %.

In the case of a thermal treatment, i.e. heating beyond Tg, the process may be continued until a fluorescence value comparable to the one obtained by UV irradiation is obtained.

In preferred embodiments of the method and article, the polymer is a hydrophilic polymer and/or has a water absorption capacity of 5 wt.-% - 50 wt.-% at a temperature of 25 °C (determined according to DIN EN ISO 18369-4:2018-04, Section 4.6). Preferably, the water absorption capacity is 20 wt.-% - 40 wt.-% at a temperature of 25 °C. The water absorption capacity may be at least 5 wt.-% or at least 10 wt.-% or at least 15 wt.-% or at least 20 wt.-% at a temperature of 25 °C. The water absorption capacity may be at most 50 wt.-% or at most 45 wt.-% or at most 40 wt.-% or at most 35 wt.-% at a temperature of 25 °C.

On the one hand, the hydrophilic polymers, in particular with such a water absorption capacity, are better biocompatible than the hydrophobic polymers. And on the other hand, they are the polymers which are most responsive to the method, i.e. they easily develop fluorescent structures and the reduction in calcification is more pronounced.

Preferably, the polymer of the method and article is a homopolymer or copolymer of (meth)acrylates, acrylamides and/or alkylene glycols.

Most preferably, the polymer of the method and article is a homopolymer or copolymer of monomers selected from the group comprising methyl methacrylate (MMA), 2-hydroxyethyl methacrylate (HEMA), 2-hydroxyethyl acrylate (HEA), butyl acrylate (BA), lauryl acrylate (LA), ethoxyethyl methacrylate (EOEMA), methacrylic acid (MAA), acrylamide (AA), ethylene glycol dimethacrylate (EGDMA), diethylene glycol bismethacrylate (DEGMA), butanediol diacrylate (BDDA), N,N'-methylene bis(acrylamide) (MBA), ethylene glycol, propylene glycol, poly(ethylene glycol) phenyl ether acrylate (PEGPEA), and 2-phenylphenoxyethyl acrylate (OPPEA). The copolymers may either comprise only the aforementioned monomers or also other monomers wherein the aforementioned monomers form more than 50 mol-% of the copolymer. These particular hydrophilic and hydrophobic homopolymers and copolymers are most effectively treated with the method according to the invention. The method according to the invention achieves for example for all hydrophilic polymers based on MMA or its derivatives, e. g. HEMA, a reduction of the risk of calcifications by at least 50 %.

When used in the method according to the invention, the polymer article may have the shape of an intraocular lens, a "button" or of a preform of an intraocular lens. Generating the fluorescent structure may conveniently be performed on the final product, i.e. as an additional final step to the normal production process known in the prior art. Hence, the normal workflow will not have to be changed and only defect free lenses will be treated. However, also the preforms of IOLs may effectively be treated before shaping the lenses.

Most preferably, the step of generating the fluorescent structure comprises irradiating the polymer with UV radiation and/or heating the polymer above its glass transition temperature Tg. The glass transition temperature Tg may be determined according to DIN 51007:2019-04. Preferably, the UV radiation has a wavelength of 250 nm - 380 nm, more preferably of 265 nm - 350 nm or of 265 nm - 315 nm. Preferably, the heat treatment is conducted moderately above Tg, particularly in the range of (Tg + 1 °C) - (Tg + 130 °C) or more preferably in the range of (Tg + 1 °C) - (Tg + 100 °C) or (Tg + 1 °C) - (Tg + 70 °C) or (Tg + 5 °C) - (Tg + 50 °C).

The fluorescent structure may be generated by irradiation or heating above Tg alone or in combination. When irradiating the polymer article, the fluorescence is generated only in the surface layer of the polymer extending 300 µm into the bulk of the article. Only after increased irradiation times the fluorescence effect spreads up to about 200 µm into the bulk. For the most commonly used irradiation times, the effect is limited to a depth of about 70 µm - 100 µm, which is still sufficient to achieve the desired reduction of the calcification. When heating the polymer, the fluorescent structure is generated uniformly within the whole bulk. Both effects can be used to tailor the desired effect. The combination may help to reduce treatment time. The combination may also be used to tailor the surface effects in relation to the bulk effects.

In order to be able to fold and unfold the lenses during the insertion process into the patient's eye, the polymer should have a certain range of hardness. If the material is too stiff, the lens will be difficult to fold, whereas insufficient stiffness impedes correct unfolding of the lens in the eye. If the material is out of the required range, the lens can be damaged or even break during insertion. Typically, the VLRH (very low rubber hardness) hardness measured according to DIN ISO 27588:2014-12 of a photochemically treated hydrophilic polymer is very little and below the analytical resolution. For thermally treated polymers VLRH hardness increases by about 1-2 units VLRH, e.g. from 88.5 VLRH - 89 VLRH to 90 VLRH - 90.5 VLRH for a typical hydrophilic polymer tested. In an embodiment, the polymer has a hardness of 80 VLRH - 100 VLRH, preferably of 85 VLRH - 95 VLRH.

The hardness increase is small and does not affect the folding of a lens made of a treated material.

Since the generation of the fluorescent structure also has an impact on other properties of the polymer, like hydrophobicity and hardness, it is, hence, important to choose between the irradiation treatment and the heat treatment also based on the desired change of these properties in bulk or only on the surface of the polymer.

For treatment, the polymer lenses or preforms are mounted in a holder and irradiated by a UV lamp, e.g. mercury lamps or UV-LEDs, and/or are heated beyond their glass transition temperature for a sufficient time until a blue fluorescence develops. This can either be controlled visually or automated by sensors. Then the lenses or preforms are rotated and irradiated a second time from the backside. Alternatively, they may be simultaneously irradiated from both sides. In the case of the thermal treatment, this step is not required due to the bulk effect.

In some embodiments, irradiating and/or heating is carried out under reduced pressure, i.e. at a pressure below ambient pressure, preferably at an absolute pressure of ≤ 900 hPa. The absolute pressure may for example be ≤ 900 hPa or ≤ 800 hPa or ≤ 700 hPa or ≤ 600 hPa or ≤ 500 hPa or ≤ 400 hPa or ≤ 300 hPa or ≤ 200 hPa or ≤ 100 hPa or ≤ 50 hPa or ≤ 10 hPa. The absolute pressure may for example be ≥ 800 hPa or ≥ 700 hPa or ≥ 600 hPa or ≥ 500 hPa or ≥ 400 hPa or ≥ 300 hPa or ≥ 200 hPa or ≥ 100 hPa or ≥ 50 hPa or ≥ 10 hPa or ≥ 1 hPa or 0.1 hPa or ≥ 0.01 hPa or ≥ 0.001 hPa. The absolute pressure may for example be 0.001 hPa - 900 hPa or 0.001 hPa - 800 hPa or 0.001 hPa - 700 hPa or 0.001 hPa - 600 hPa or 0.001 hPa - 500 hPa or 0.001 hPa - 400 hPa or 0.001 hPa - 300 hPa or 0.001 hPa - 200 hPa or 0.001 hPa - 100 hPa or 0.001 hPa - 50 hPa or 0.001 hPa - 10 hPa or 0.001 hPa - 1 hPa or 0.001 hPa - 0.1 hPa or 0.001 hPa - 0.01 hPa. The absolute pressure may for example be 0.01 hPa - 900 hPa or 0.01 hPa - 800 hPa or 0.01 hPa - 700 hPa or 0.01 hPa - 600 hPa or 0.01 hPa - 500 hPa or 0.01 hPa - 400 hPa or 0.01 hPa - 300 hPa or 0.01 hPa - 200 hPa or 0.01 hPa - 100 hPa or 0.01 hPa - 50 hPa or 0.01 hPa - 10 hPa or 0.01 hPa - 1 hPa or 0.01 hPa - 0.1 hPa. The absolute pressure may for example be 0.1 hPa - 900 hPa or 0.1 hPa - 800 hPa or 0.1 hPa - 700 hPa or 0.1 hPa - 600 hPa or 0.1 hPa - 500 hPa or 0.1 hPa - 400 hPa or 0.1 hPa - 300 hPa or 0.1 hPa - 200 hPa or 0.1 hPa - 100 hPa or 0.1 hPa - 50 hPa or 0.1 hPa - 10 hPa or 0.1 hPa - 1 hPa. The absolute pressure may for example be 1 hPa - 900 hPa or 1 hPa - 800 hPa or 1 hPa - 700 hPa or 1 hPa - 600 hPa or 1 hPa - 500 hPa or 1 hPa - 400 hPa or 1 hPa - 300 hPa or 1 hPa - 200 hPa or 1 hPa - 100 hPa or 1 hPa - 50 hPa or 1 hPa - 10 hPa. The absolute pressure may for example be 10 hPa - 900 hPa or 10 hPa - 800 hPa or 10 hPa - 700 hPa or 10 hPa - 600 hPa or 10 hPa - 500 hPa or 10 hPa - 400 hPa or 10 hPa - 300 hPa or 10 hPa - 200 hPa or 10 hPa - 100 hPa or 10 hPa - 50 hPa. The absolute pressure may for example be 50 hPa - 900 hPa or 50 hPa - 800 hPa or 50 hPa - 700 hPa or 50 hPa - 600 hPa or 50 hPa - 500 hPa or 50 hPa - 400 hPa or 50 hPa - 300 hPa or 50 hPa - 200 hPa or 50 hPa - 100 hPa. The absolute pressure may for example be 100 hPa - 900 hPa or 100 hPa - 800 hPa or 100 hPa - 700 hPa or 100 hPa - 600 hPa or 100 hPa - 500 hPa or 100 hPa - 400 hPa or 100 hPa - 300 hPa or 100 hPa - 200 hPa. The absolute pressure may for example be 200 hPa - 900 hPa or 200 hPa - 800 hPa or 200 hPa - 700 hPa or 200 hPa - 600 hPa or 200 hPa - 500 hPa or 200 hPa - 400 hPa or 200 hPa - 300 hPa. The absolute pressure may for example be 300 hPa - 900 hPa or 300 hPa - 800 hPa or 300 hPa - 700 hPa or 300 hPa - 600 hPa or 300 hPa - 500 hPa or 300 hPa - 400 hPa. The absolute pressure may for example be 400 hPa - 900 hPa or 400 hPa - 800 hPa or 400 hPa - 700 hPa or 400 hPa - 600 hPa or 400 hPa - 500 hPa. The absolute pressure may for example be 500 hPa - 900 hPa or 500 hPa - 800 hPa or 500 hPa - 700 hPa or 500 hPa - 600 hPa. The absolute pressure may for example be 600 hPa - 900 hPa or 600 hPa - 800 hPa or 600 hPa - 700 hPa. The absolute pressure may for example be 700 hPa - 900 hPa or 700 hPa - 800 hPa. The absolute pressure may for example be 800 hPa - 900 hPa. Particularly preferred ranges of the absolute pressure are for example 0.001 hPa - 900 hPa, 0.001 hPa - 200 hPa, 0.001 hPa - 100 hPa, or 0.01 hPa - 10 hPa. A reduced pressure is advantageous for removing low molecular products which may possibly be generated during the treatment.

Further, it is advantageous if prior to the step of generating the fluorescent structure, the content of residual monomers in the polymer is low or reduced. Preferably, it is reduced to a concentration of < 1 wt.-% relative to the total amount of the polymer, more preferably to a concentration of < 0.9 wt.-% or < 0.8 wt.-% or < 0.7 wt.-% or < 0.6 wt.-% or < 0.5 wt.-% or < 0.4 wt.-% or < 0.3 wt.-% or < 0.2 wt.-% or < 0.1 wt.-%. It may be reduced to a concentration of > 0.9 wt.-% or > 0.8 wt.-% or > 0.7 wt.-% or > 0.6 wt.-% or > 0.5 wt.-% or > 0.4 wt.-% or > 0.3 wt.-% or > 0.2 wt.-% or > 0.1 wt.-% or > 0.01 wt.-% or > 0.001 wt.-% or > 0.0001 wt.-%. It may be reduced to a concentration of 0.0001 wt.-% - < 1 wt.-% or 0.0001 wt.-% - < 0.9 wt.-% or 0.0001 wt.-% - < 0.8 wt.-% or 0.0001 wt.-% - < 0.7 wt.-% or 0.0001 wt.-% - < 0.6 wt.-% or 0.0001 wt.-% - < 0.5 wt.-% or 0.0001 wt.-% - < 0.4 wt.-% or 0.0001 wt.-% - < 0.3 wt.-% or 0.0001 wt.-% - < 0.2 wt.-% or 0.0001 wt.-% - < 0.1 wt.-% or 0.0001 wt.-% - < 0.01 wt.-% or 0.0001 wt.-% - < 0.001 wt.-%. It may be reduced to a concentration of 0.001 wt.-% - < 1 wt.-% or 0.001 wt.-% - < 0.9 wt.-% or 0.001 wt.-% - < 0.8 wt.-% or 0.001 wt.-% - < 0.7 wt.-% or 0.001 wt.-% - < 0.6 wt.-% or 0.001 wt.-% - < 0.5 wt.-% or 0.001 wt.-% - < 0.4 wt.-% or 0.001 wt.-% - < 0.3 wt.-% or 0.001 wt.-% - < 0.2 wt.-% or 0.001 wt.-% - < 0.1 wt.-% or 0.001 wt.-% - < 0.01 wt.-%. It may be reduced to a concentration of 0.01 wt.-% - < 1 wt.-% or 0.01 wt.-% - < 0.9 wt.-% or 0.01 wt.-% - < 0.8 wt.-% or 0.01 wt.-% - < 0.7 wt.-% or 0.01 wt.-% - < 0.6 wt.-% or 0.01 wt.-% - < 0.5 wt.-% or 0.01 wt.-% - < 0.4 wt.-% or 0.01 wt.-% - < 0.3 wt.-% or 0.01 wt.-% - < 0.2 wt.-% or 0.01 wt.-% - < 0.1 wt.-%. It may be reduced to a concentration of 0.1 wt.-% - < 1 wt.-% or 0.1 wt.-% - < 0.9 wt.-% or 0.1 wt.-% - < 0.8 wt.-% or 0.1 wt.-% - < 0.7 wt.-% or 0.1 wt.-% - < 0.6 wt.-% or 0.1 wt.-% - < 0.5 wt.-% or 0.1 wt.-% - < 0.4 wt.-% or 0.1 wt.-% - < 0.3 wt.-% or 0.1 wt.-% - < 0.2 wt.-%. It may be reduced to a concentration of 0.2 wt.-% - < 1 wt.-% or 0.2 wt.-% - < 0.9 wt.-% or 0.2 wt.-% - < 0.8 wt.-% or 0.2 wt.-% - < 0.7 wt.-% or 0.2 wt.-% - < 0.6 wt.-% or 0.2 wt.-% - < 0.5 wt.-% or 0.2 wt.-% - < 0.4 wt.-% or 0.2 wt.-% - < 0.3 wt.-%. It may be reduced to a concentration of 0.3 wt.-% - < 1 wt.-% or 0.3 wt.-% - < 0.9 wt.-% or 0.3 wt.-% - < 0.8 wt.-% or 0.3 wt.-% - < 0.7 wt.-% or 0.3 wt.-% - < 0.6 wt.-% or 0.3 wt.-% - < 0.5 wt.-% or 0.3 wt.-% - < 0.4 wt.-%. It may be reduced to a concentration of 0.4 wt.-% - < 1 wt.-% or 0.4 wt.-% - < 0.9 wt.-% or 0.4 wt.-% - < 0.8 wt.-% or 0.4 wt.-% - < 0.7 wt.-% or 0.4 wt.-% - < 0.6 wt.-% or 0.4 wt.-% - < 0.5 wt.-%. It may be reduced to a concentration of 0.5 wt.-% - < 1 wt.-% or 0.5 wt.-% - < 0.9 wt.-% or 0.5 wt.-% - < 0.8 wt.-% or 0.5 wt.-% - < 0.7 wt.-% or 0.5 wt.-% - < 0.6 wt.-%. It may be reduced to a concentration of 0.6 wt.-% - < 1 wt.-% or 0.6 wt.-% - < 0.9 wt.-% or 0.6 wt.-% - < 0.8 wt.-% or 0.6 wt.-% - < 0.7 wt.-%. It may be reduced to a concentration of 0.7 wt.-% - < 1 wt.-% or 0.7 wt.-% - < 0.9 wt.-% or 0.7 wt.-% - < 0.8 wt.-%. It may be reduced to a concentration of 0.8 wt.-% - < 1 wt.-% or 0.8 wt.-% - < 0.9 wt.-%. It may be reduced to a concentration of 0.9 wt.-% - < 1 wt.-%. Particularly preferred ranges of the concentration are for example 0.0001 wt.-% - < 0.9 wt.-%, 0.001 wt.-% - < 0.7 wt.-%, or 0.001 wt.-% - < 0.5 wt.-%. Particularly preferably the content of residual monomers in the polymer is reduced as far as possible. The optional removal of residual monomers facilitates the formation of the fluorescent structures because there is no competitive reaction of the monomers with the generated structures.

In preferred embodiments, the method comprises reducing the content of residual monomers in the polymer. The reduction of the content of residual monomers may preferably be effected by means of extraction or applying a reduced pressure, i.e. a pressure below ambient pressure, preferably an absolute pressure of ≤ 900 hPa. The absolute pressure may for example be ≤ 900 hPa or ≤ 800 hPa or ≤ 700 hPa or ≤ 600 hPa or ≤ 500 hPa or ≤ 400 hPa or ≤ 300 hPa or ≤ 200 hPa or ≤ 100 hPa or ≤ 50 hPa or ≤ 10 hPa or ≤ 1 hPa. The absolute pressure may for example be ≥ 800 hPa or ≥ 700 hPa or ≥ 600 hPa or ≥ 500 hPa or ≥ 400 hPa or ≥ 300 hPa or ≥ 200 hPa or ≥ 100 hPa or ≥ 50 hPa or ≥ 10 hPa or ≥ 1 hPa or ≥ 0.1 hPa or ≥ 0.01 hPa or ≥ 0.001 hPa. The absolute pressure may for example be 0.001 hPa - 900 hPa or 0.001 hPa - 800 hPa or 0.001 hPa - 700 hPa or 0.001 hPa - 600 hPa or 0.001 hPa - 500 hPa or 0.001 hPa - 400 hPa or 0.001 hPa - 300 hPa or 0.001 hPa - 200 hPa or 0.001 hPa - 100 hPa or 0.001 hPa - 50 hPa or 0.001 hPa - 10 hPa or 0.001 hPa - 1 hPa or 0.001 hPa - 0.1 hPa or 0.001 hPa - 0.01 hPa. The absolute pressure may for example be 0.01 hPa - 900 hPa or 0.01 hPa - 800 hPa or 0.01 hPa - 700 hPa or 0.01 hPa - 600 hPa or 0.01 hPa - 500 hPa or 0.01 hPa - 400 hPa or 0.01 hPa - 300 hPa or 0.01 hPa - 200 hPa or 0.01 hPa - 100 hPa or 0.01 hPa - 50 hPa or 0.01 hPa - 10 hPa or 0.01 hPa - 1 hPa or 0.01 hPa - 0.1 hPa. The absolute pressure may for example be 0.1 hPa - 900 hPa or 0.1 hPa - 800 hPa or 0.1 hPa - 700 hPa or 0.1 hPa - 600 hPa or 0.1 hPa - 500 hPa or 0.1 hPa - 400 hPa or 0.1 hPa - 300 hPa or 0.1 hPa - 200 hPa or 0.1 hPa - 100 hPa or 0.1 hPa - 50 hPa or 0.1 hPa - 10 hPa or 0.1 hPa - 1 hPa. The absolute pressure may for example be 1 hPa - 900 hPa or 1 hPa - 800 hPa or 1 hPa - 700 hPa or 1 hPa - 600 hPa or 1 hPa - 500 hPa or 1 hPa - 400 hPa or 1 hPa - 300 hPa or 1 hPa - 200 hPa or 1 hPa - 100 hPa or 1 hPa - 50 hPa or 1 hPa - 10 hPa. The absolute pressure may for example be 10 hPa - 900 hPa or 10 hPa - 800 hPa or 10 hPa - 700 hPa or 10 hPa - 600 hPa or 10 hPa - 500 hPa or 10 hPa - 400 hPa or 10 hPa - 300 hPa or 10 hPa - 200 hPa or 10 hPa - 100 hPa or 10 hPa - 50 hPa. The absolute pressure may for example be 50 hPa - 900 hPa or 50 hPa - 800 hPa or 50 hPa - 700 hPa or 50 hPa - 600 hPa or 50 hPa - 500 hPa or 50 hPa - 400 hPa or 50 hPa - 300 hPa or 50 hPa - 200 hPa or 50 hPa - 100 hPa. The absolute pressure may for example be 100 hPa - 900 hPa or 100 hPa - 800 hPa or 100 hPa - 700 hPa or 100 hPa - 600 hPa or 100 hPa - 500 hPa or 100 hPa - 400 hPa or 100 hPa - 300 hPa or 100 hPa - 200 hPa. The absolute pressure may for example be 200 hPa - 900 hPa or 200 hPa - 800 hPa or 200 hPa - 700 hPa or 200 hPa - 600 hPa or 200 hPa - 500 hPa or 200 hPa - 400 hPa or 200 hPa - 300 hPa. The absolute pressure may for example be 300 hPa - 900 hPa or 300 hPa - 800 hPa or 300 hPa - 700 hPa or 300 hPa - 600 hPa or 300 hPa - 500 hPa or 300 hPa - 400 hPa. The absolute pressure may for example be 400 hPa - 900 hPa or 400 hPa - 800 hPa or 400 hPa - 700 hPa or 400 hPa - 600 hPa or 400 hPa - 500 hPa. The absolute pressure may for example be 500 hPa - 900 hPa or 500 hPa - 800 hPa or 500 hPa - 700 hPa or 500 hPa - 600 hPa. The absolute pressure may for example be 600 hPa - 900 hPa or 600 hPa - 800 hPa or 600 hPa - 700 hPa. The absolute pressure may for example be 700 hPa - 900 hPa or 700 hPa - 800 hPa. The absolute pressure may for example be 800 hPa - 900 hPa. Particularly preferred ranges of the absolute pressure are for example 0.001 hPa - 900 hPa, 0.001 hPa - 10 hPa, 0.001 hPa - 1 hPa, or 0.01 hPa - 1 hPa.

In embodiments, the hydrophilicity of the surface of the polymer as determined by contact angle measurement may be higher as compared to before treatment, such that the contact angle is lower. Preferably, the hydrophilicity is increased by the treatment such that the contact angle is decreased by at least 5°. The contact angle measurements may be conducted according to DIN 55660-2:2011-12. The contact angle may be decreased by more than 10 % as compared to before treatment, preferably by up to 17.5 % or by up to 25 % or by up to 30 %. The contact angle may be decreased by 10 %-15 % or by 10 %-17.5 % or by 10 %-20 % or by 10 %-22.5 % or by 10 %-25 % or by 10 %-27.5 % or by 10 %-30 %. For example, the contact angle may be reduced from 75.4° ± 3.9° to 58.5° ± 5.4° by UV irradiation, which means a reduction of 22.5 %. In another example, the contact angle is reduced from 90° - 95° to 65° - 70°. The change in contact angle is dependent on the treatment time.

In particularly preferred embodiments, the fluorescent structure fluoresces in the blue section of the visible spectrum. Preferably, the fluorescence is at a wavelength of 430 nm - 490 nm. It has been found that structures fluorescing at these wavelengths are particularly effective in reducing the *in vivo* calcification of the lenses.

Another aspect of the present invention is the use of an article treated by a method according to the invention in an ophthalmic medical aid, in particular in intraocular lenses. These ophthalmic medical aids will benefit from the significantly reduced *in vivo* calcification of the polymers according to the invention.

A further aspect of the present invention is an article comprising one or more polymers, optionally shaped as an optical lens, an intraocular lens, or a "button" or a preform of an optical or intraocular lens, wherein one or more of the polymers comprises an acrylic structural motive, comprising a fluorescent structure.

Preferably, the fluorescent structure is non-extractable from the polymer article, is obtainable by intramolecularly and/or intermolecularly condensing substituents and/or structural units of a polymer chain of the polymer, and/or comprises a lactone structure.

The article preferably has a refractive index measured at 589 nm and 20 °C between 1.50 and 1.59 in the dry state and/or between 1.45 and 1.47 in the hydrated with deionized water state. The "dry state" is referring to the state after drying at 50 °C in ambient air until no further weight reduction occurs. The "hydrated with deionized water" state is referring to the state after immersing the article in deionized water at 25 °C until no further weight increase occurs.

In embodiments, in the article, comprising a bulk volume and a surface layer surrounding the bulk volume, the surface layer extending 300 µm into the article, the fluorescent structure is present (i) in bulk volume, (ii) in the bulk volume and in the surface layer, (iii) in the surface layer, or (iv) in the surface layer and not in the bulk volume.

In an example of the method, the reduction of the content of residual monomers is effected by means of applying an absolute pressure of 0.001 hPa - 900 hPa, the content of residual monomers in the polymer is reduced to a concentration of 0.0001 wt.-% - < 0.9 wt.-%, irradiating and/or heating is carried out at an absolute pressure of 0.001 hPa - 900 hPa, and the reduction of the calcification propensity amounts to 50 % - 100 %.

In another example of the method, the reduction of the content of residual monomers is effected by means of applying an absolute pressure of 0.001 hPa - 10 hPa, the content of residual monomers in the polymer is reduced to a concentration of 0.001 wt.-% - < 0.7 wt.-%, irradiating and/or heating is carried out at an absolute pressure of 0.001 hPa - 200 hPa, and the reduction of the calcification propensity amounts to 60 % - 95 %.

In another example of the method, the reduction of the content of residual monomers is effected by means of applying an absolute pressure of 0.001 hPa - 1 hPa, the content of residual monomers in the polymer is reduced to a concentration of 0.001 wt.-% - < 0.5 wt.-%, irradiating and/or heating is carried out at an absolute pressure of 0.001 hPa - 100 hPa, and the reduction of the calcification propensity amounts to 70 % - 90 %.

In another example of the method, the reduction of the content of residual monomers is effected by means of applying an absolute pressure of 0.01 hPa - 1 hPa, the content of residual monomers in the polymer is reduced to a concentration of 0.001 wt.-% - < 0.5 wt.-%, irradiating and/or heating is carried out at an absolute pressure of 0.01 hPa - 10 hPa, and the reduction of the calcification propensity amounts to 70 % - 90 %.

### Brief description of the figures

- **Figure 1**: shows photos of two analyzed explanted intraocular lenses.
- **Figure 2**: shows micrographs of the surface and a cross-section of the lenses of Figure 1.
- **Figure 3**: shows a lens "button" whose left half is untreated and whose right half has been irradiated according to the present invention to show a blue fluorescence.
- **Figure 4**: shows two samples of lens "buttons" of Figure 3 after a 60 day incubation time in solution L3.
- **Figure 5**: shows pictures of lens "buttons" of different compositions which have been irradiated according to the present invention on their right halves (upper row) and after a 60 day incubation time in solution L3 (lower row).
- **Figure 6**: shows a picture of the development of the fluorescence of a lens "button" during a 24 hour treatment at 200 °C and 15 hPa and the half irradiated lens "button" of Figure 3 as a reference.
- **Figure 7**: shows two sets of lens "buttons" after an incubation time of 226 days in solution L3, wherein the first set in the upper row is an untreated reference and the second set in the lower row has been irradiated according to the invention for 72 hours on each side with an intensity of 500 W/m² through quartz glass.
- **Figure 8**: shows three sets of lens "buttons" after an incubation time of 129 days in solution L3, wherein the first set in the upper row is an untreated reference, the second set in the middle row has been irradiated according to the invention and the third set in the lower row has been thermally treated according to the invention.
- **Figure 9**: shows a cross section through an irradiated IOL and its blue fluorescence.
- **Figure 10**: shows a side view of an irradiated polymer without UV absorber and its blue fluorescence.

### Examples

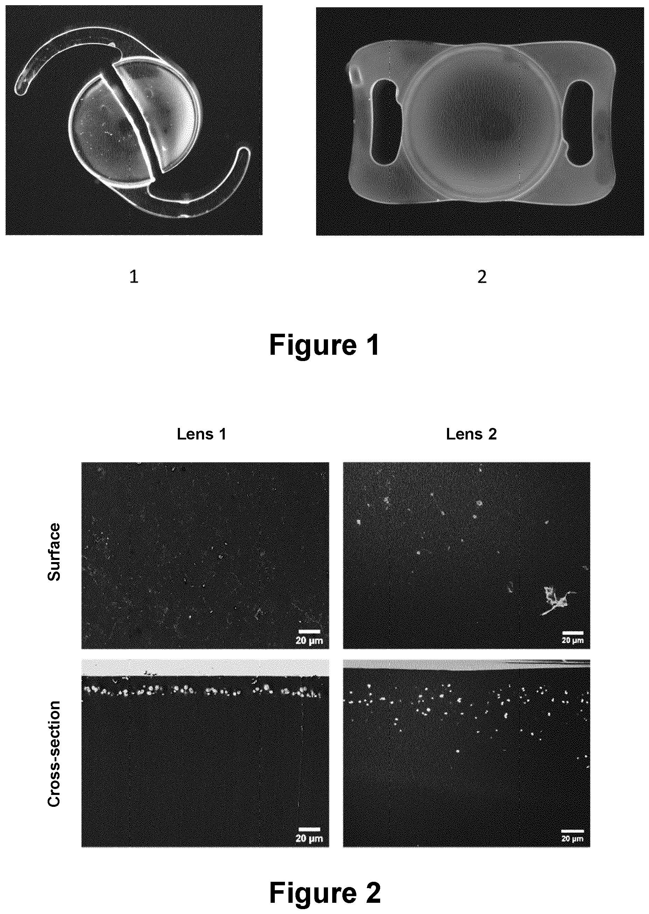

A set of two different types of explanted intraocular lenses has been analyzed regarding the calcification effect. **Figure 1** shows photos of these IOLs.

From these IOLs, micrographs of their surface and cross-sections created by microtome cutting have been prepared for inspection of the calcification structure and position. **Figure 2** shows pictures of the micrographs of their surface and a cross-section. One can clearly see that the calcific deposits are not only located on the surface but also to a certain extent below the surface (up to about 60-70 µm).

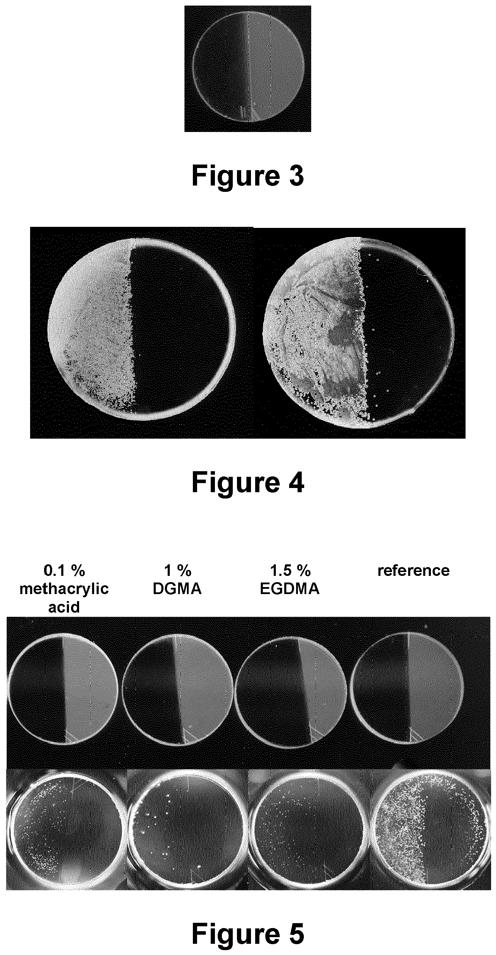

An example of a polymer which has been treated with a method according to the invention by UV irradiation is shown in **Figure 3****.** A lens "button" whose left half has been covered by aluminum foil has been irradiated with 500 W/cm² in the range from 300 nm to 800 nm for 12 days. In Figure 3, the difference between the untreated left part and the treated right part is clearly visible. The treated half shows a blue fluorescence with a maximum between 430 nm and 490 nm.

In order to study the effectiveness of the treatment against calcification, two of these half-treated samples have been used in an *in vitro* calcification test wherein they were stored for 60 days in an incubator at 35 °C in solution L3 as described above. **Figure 4** shows the two samples after the incubation time. While the untreated halves are almost completely covered with calcific deposits, the treated halves are nearly free of any deposits.

Another set of four intraocular lens "buttons", which is shown in **Figure 5**, has been subjected to the same test conditions. The four "buttons" differ in their added impurities. In this experiment, the effect of typical impurities, which may be present in the polymer, on the generation of the fluorescing structure has been investigated. The samples 1-3 contain 0.1 wt.-% MA, 1 wt.-% DGMA and 1.5 wt.-% EGDMA as examples of monomer and crosslinker residues while sample 4 is the pure reference. The pictures show in the upper row the lens "buttons" after half-sided irradiation according to the present invention. Their right halves accordingly show a blueish fluorescence. In the lower row, the same "buttons" are shown after the 60 day incubation time in solution L3. Also in this comparison, the halves treated according to the invention are almost free from deposits while the untreated sides show various degrees of deposits. The result demonstrates that impurities do not have a negative impact on the generation of the fluorescence or the calcification reduction effect.

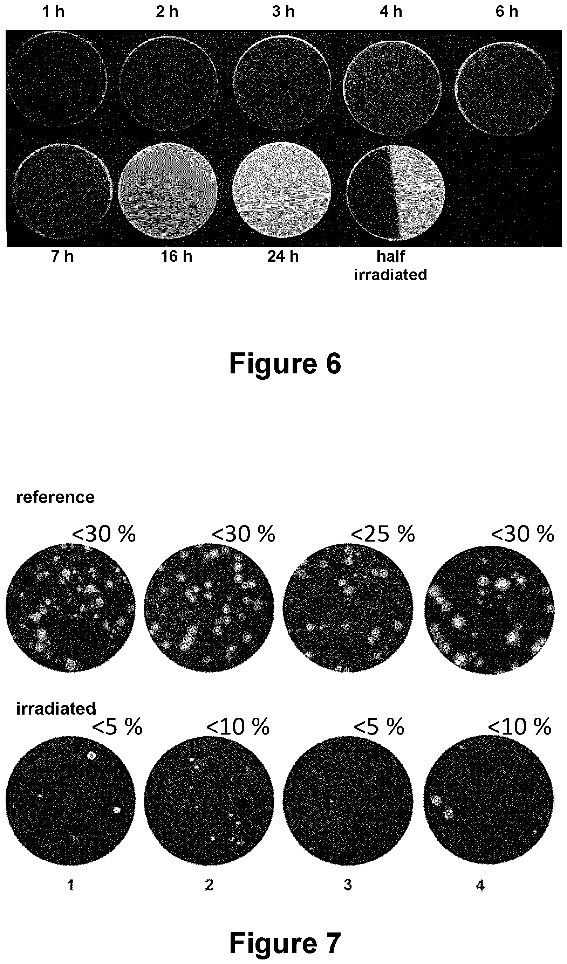

The thermal treatment variant of the method according to the invention has been demonstrated in **Figure 6**, which shows a picture of the development of the fluorescence of a lens "button" during a 24 hour treatment at 200 °C and 15 hPa and the half irradiated lens "button" of Figure 3 as a reference. After a treatment time of about 16 hours, a fluorescence matching that of the UV irradiation test of Figure 4 is achieved.

The long term effect of the method has been investigated in an incubation test of 226 days in solution L3. Two sets of IOL "buttons", an untreated reference set and a set irradiated according to the invention for 72 hours on each side with an intensity of 500 W/m² through quartz glass, have been compared. Each set comprises four identical samples. **Figure** 7 shows the two sets after the incubation time, wherein the upper row is the untreated reference and the lower row is the set according to the invention. The reduction of the calcification is evident even for this long-term test. While the set of reference samples shows a calcification propensity of (< 30 %, < 30 %, < 25 %, < 30 %), the set of irradiated samples has calcification propensities of only (< 5 %, < 10 %, < 5 %, < 10 %). This clearly demonstrates that the effect created by the invention is a permanent one.

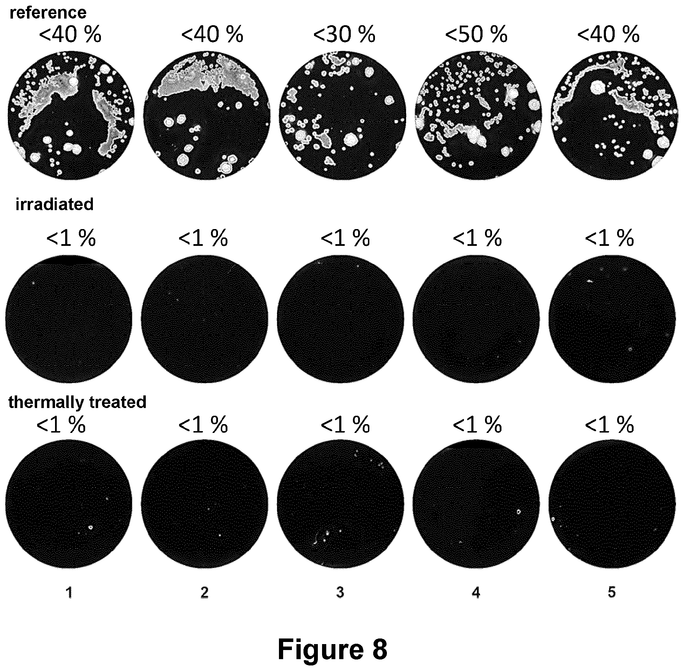

A comparison between the UV treatment and the thermal treatment according to the invention has been conducted in an incubation test of 129 days in solution L3. The main components of the used polymer are methyl methacrylate (MMA) and hydroxyethyl methacrylate (HEMA). **Figure** 8 shows the image-stack images of samples obtained according to the measurement method described above. The diameter of the sample images is about 10 mm. Shown are three sets of lens "buttons" after the incubation time, wherein the first set in the upper row is an untreated reference, the second set in the middle row is the one irradiated by UV light and the third set in the lower row is the thermally treated one. The irradiation has been made with a UV lamp of 290-310 nm and 1.2 mW/cm² for five days or with an LED of 300 nm and 7.14 mW/cm² for three days. Thermal treatment has been made at 200 °C and 15 hPa for 17 hours. Fluorescence excitation was performed with 365 ± 10 nm. While the set of reference samples shows a calcification propensity of (< 40 %, < 40 %, < 30 %, < 50 %, < 40 %), the sets of treated samples all have calcification propensities of only < 1 %. The irradiated samples show an almost complete suppression of calcification, while the thermally treated samples show a high degree of calcification suppression.

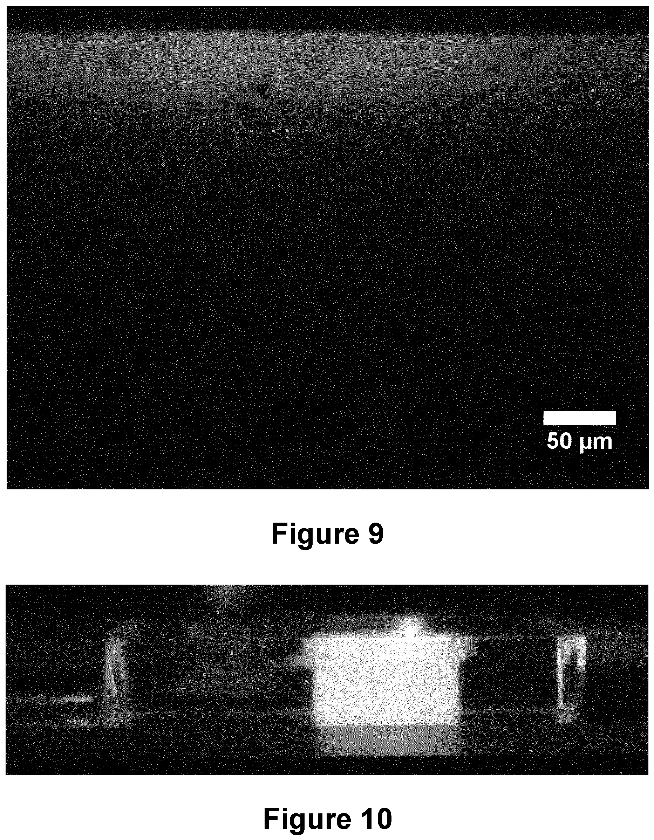

In order to demonstrate the depth of the effect which can be achieved, a comparison between a standard polymer used for IOLs including an UV absorber and the same polymer without UV absorber has been made. Both samples have been irradiated by UV light to create a fluorescence. **Figure** 9 shows a cross section through an irradiated IOL made from the standard polymer. The thickness of the blue fluorescent layer is about 70 µm. Depending on the intensity and duration of the radiation, the fluorescent layer can be up to 200 µm. **Figure 10** shows a side view of an irradiated button made from the same polymer but without UV absorber and having a thickness of 3 mm. As can be seen, the fluorescence generated by the UV irradiation extends vertically through the whole button. The whole sample was photochemically irradiated. To visualize the fluorescence spanning the whole material thickness a smaller excitation beam was used. Only exactly the excited zone in the middle of the button fluoresces.

## Claims

1. Method for reducing the calcification propensity of an article, optionally shaped as an optical lens, an intraocular lens, or a button or a preform of an optical or intraocular lens, the method comprising
generating one or more fluorescent structures in or on the article,
wherein the article comprises one or more polymers,
wherein one or more of the polymers comprises an acrylic structural motive.

2. Method according to claim 1, wherein generating one or more fluorescent structures comprises
partially or completely irradiating the article with UV radiation, preferably having a wavelength of 250 nm - 380 nm, and/or
heating the article above the glass transition temperature Tg of the polymer.

3. Method according to claim 1 or 2, wherein generating one or more fluorescent structures comprises
generating a fluorescent structure in the polymer, particularly by intramolecularly and/or intermolecularly condensing substituents and/or structural units of the polymer chain.

4. Method according to one or more of claims 1 to 3, wherein
calcification propensity of the polymer is reduced by at least 50 % as compared to before treatment, and/or
generating one or more fluorescent structures is performed such that the fluorescence intensity of the article is at least 80 % of the maximum intensity achievable in the article by UV irradiation.

5. Method according one or more of claims 2 to 4, wherein heating is carried out at a pressure below ambient pressure, preferably at an absolute pressure of ≤ 900 hPa.

6. Method according to one of the preceding claims, wherein prior to the step of generating the fluorescent structure, the content of residual monomers in the polymer is low or reduced, preferably to a concentration of < 1 wt.-% relative to the total amount of the polymer.

7. Method according to one of the preceding claims, wherein the method comprises
reducing the content of residual monomers in the polymer, preferably by means of extraction or applying a pressure below ambient pressure, preferably an absolute pressure of ≤ 900 hPa.

8. Method according to one of the preceding claims, wherein
- the hydrophilicity of the surface of the polymer as determined by contact angle measurement according to DIN 55660-2:2011-12 is higher as compared to before treatment, such that the contact angle is lower, preferably by at least 5°, and/or
- the VLRH hardness as determined according to DIN ISO 27588:2014-12 of the surface of the polymer is higher as compared to before treatment, preferably by not more than 10 %.

9. Article comprising one or more polymers, optionally shaped as an optical lens, an intraocular lens, or a button or a preform of an optical or intraocular lens,
wherein one or more of the polymers comprises an acrylic structural motive,
comprising a fluorescent structure.

10. Article according to claim 9 or method according to one of claims 1 to 8, wherein the fluorescent structure
- is non-extractable from the polymer article, and/or
- is obtainable by intramolecularly and/or intermolecularly condensing substituents and/or structural units of a polymer chain of the polymer, and/or
- comprises a lactone structure, and/or
- fluoresces in the blue section of the visible spectrum, preferably at a wavelength of 430 nm - 490 nm.

11. Article according to claim 9 or 10,
- having a refractive index measured at 589 nm and 20 °C of between 1.50 and 1.59 in the dry state and/or between 1.45 and 1.47 in the hydrated with deionized water state, and/or
- wherein the fluorescence intensity of the article is at least 80 % of the maximum intensity achievable in the article by UV irradiation, and/or
- having a calcification propensity of less than 10 %.

12. Article according to one or more of claims 9 to 11, comprising a bulk volume and a surface layer surrounding the bulk volume, the surface layer extending 300 µm into the article, wherein the fluorescent structure is present
(i) in the bulk volume,
(ii) in the bulk volume and in the surface layer,
(iii) in the surface layer, or
(iv) in the surface layer and not in the bulk volume.

13. Article according to one or more of claims 9 to 12 or method according to claims 1 to 8, wherein
- the polymer is a hydrophilic polymer and/or has a water absorption capacity of 5 wt.-% - 50 wt.-%, preferably 20 wt.-% - 35 wt.-% at a temperature of 25 °C, and/or
- the polymer is a homopolymer or copolymer of (meth)acrylates, acrylamides and/or alkylene glycols, and/or
- the polymer is a homopolymer or copolymer of monomers selected from the group comprising methyl methacrylate (MMA), 2-hydroxyethyl methacrylate (HEMA), 2-hydroxyethyl acrylate (HEA), butyl acrylate (BA), lauryl acrylate (LA), ethoxyethyl methacrylate (EOEMA), methacrylic acid (MAA), acrylamide (AA), ethylene glycol dimethacrylate (EGDMA), diethylene glycol bismethacrylate (DEGMA), butanediol diacrylate (BDDA), N,N'-methylene bis(acrylamide) (MBA), ethylene glycol, propylene glycol, poly(ethylene glycol) phenyl ether acrylate (PEGPEA), and 2-phenylphenoxyethyl acrylate (OPPEA).

14. Article according to one or more of claims 10 to 19, obtainable by a method according to claims 1 to 9.

15. Use of an article according to claims 10 to 20, in an ophthalmic medical aid, in particular in intraocular lenses.
